# EUROPEAN PATENT APPLICATION

(11) **EP 1 553 171 A1**
(43) Date of publication of application: **13.07.2005**
(21) Application number: 03794067.3
(22) Date of filing: 25.07.2003
(51) Int. Cl.: C12N 9/18, C12N 9/48, C12P 21/02

(54) **NOVEL ENZYME FORMING PEPTIDE, MICROORGANISM PRODUCING THE SAME AND PROCESS FOR PRODUCING DIPEPTIDE USING THEM**

(30) Priority: 26.07.2002 JP 2002218956
(71) Applicant: Ajinomoto Co., Inc., Tokyo 104-8315 (JP)
(72) Inventor: YOKOZEKI, Kenzo, /o Ajinomoto Co., Inc., Kawasaki-shi, Kanagawa 210-8681 (JP); SUZUKI, Sonoko, c/o Ajinomoto Co., Inc., Kawasaki-shi, Kanagawa 210-8681 (JP); HARA, Seiichi, c/o Ajinomoto Co., Inc., Kawasaki-shi, Kanagawa 210-8681 (JP); KATAYAMA, Satoshi, c/o Ajinomoto Co., Inc., Kawasaki-shi, Kanagawa 210-8681 (JP)
(74) Representative: Strehl Schübel-Hopf & Partner
(86) International application number: PCT/JP2003/009467
(87) International publication number: WO 2004/022733

(57) **Abstract**

The present invention relates to a novel enzyme that allows peptide to be produced easily, inexpensively and at high yield without going through a complex synthesis method. More particularly, the present invention provides a novel enzyme that catalyzes a peptide-producing reaction from a carboxy component and an amine component, a microbe that produces the enzyme, and a method for inexpensive production of peptides using this enzyme or microbe. The novel enzyme that efficiently produces peptide was discovered from a newly discovered microbe belonging to the genus *Empedobacter,* and a method was found that allows peptides to be produced inexpensively and easily.

## Description

### TECHNICAL FIELD

The present invention relates to a novel enzyme that can produce a peptide easily, inexpensively and at high yield without going through a complex synthesis method. More particularly, the present invention relates to a novel enzyme that catalyzes a peptide-producing reaction from a carboxy component and an amine component, to a microbe that produces the enzyme, and a method for producing a dipeptide using the enzyme or microbe.

### BACKGROUND ART

Peptides are used in the fields of pharmaceuticals, foods and various other fields. For example, since L-alanyl-L-glutamine has higher stability and water-solubility than L-glutamine, it is widely used as a component of fluid infusion and serum-free media.

Chemical synthesis methods, which have been known as methods for producing peptides, are not always easy. Known examples of such methods include a method that uses N-benzyloxycarbonylalanine (hereinafter, "Z-alanine") and protected L-glutamine (see Bull. Chem. Soc. Jpn., 34, 739 (1961), Bull. Chem. Soc. Jpn., 35, 1966 (1962)), a method that uses Z-alanine and protected L-glutamic acid-γ-methyl ester (see Bull. Chem. Soc. Jpn., 37, 200 (1964)), a method that uses Z-alanine ester and unprotected glutamic acid (see Japanese Patent Application Laid-open Publication No. H1-96194), a method that involves synthesis of an N-(2-substituted)-propionyl glutamine derivative as an intermediate from a 2-substituted-propionyl halide as a raw material (see Patent Application Laid-open Publication No. H6-234715).

However, since all of these methods require the introduction and elimination of protecting groups or the use of an optically active intermediate, they are not considered to be adequately satisfactory in terms of their industrial advantages.

On the other hand, widely known examples of typical peptide production methods using enzymes consist of a condensation reaction that uses an N-protected and C-unprotected carboxy component and an N-unprotected, C-protected amine component (hereinafter, "Reaction 1"), and a substitution reaction that uses an N-protected, C-protected carboxy component and an N-unprotected, C-protected amine component (hereinafter, "Reaction 2"). An example of Reaction 1 is a method for producing Z-aspartylphenylalanine methyl ester from Z-aspartic acid and phenylalanine methyl ester (see Japanese Patent Application Laid-open Publication No. S53-92729), while an example of Reaction 2 is a method for producing acetylphenylalanylleucine amide from acetylphenylalanine ethyl ester and leucine amide (see Biochemical J., 163, 531 (1977)). There have been reported very few research examples of methods that use an N-unprotected, C-protected carboxy component. An example of a substitution reaction that uses an N-unprotected, C-protected carboxy component and an N-unprotected, C-protected amine component (hereinafter, "Reaction 3") is described in International Patent Publication WO 90/01555. For example, a method for producing arginylleucine amide from arginine ethyl ester and leucine amide may be mentioned of. Examples of substitution reactions that use an N-unprotected, C-protected carboxy component and an N-unprotected, C-unprotected amine component (hereinafter, "Reaction 4") are described in European Patent Publications EP 278787A1 and EP 359399B1. For example, a method for producing tyrosylalanine from tyrosine ethyl ester and alanine may be mentioned of.

### DISCLOSURE OF THE INVENTION

The most inexpensive production method among the aforementioned methods of Reactions 1 to 4 naturally falls within the class of Reaction 4, which involves the fewest protecting groups.

However, the example of Reaction 4 of the prior art (European Patent Publication EP 278787A1) had the following major problems:
(1) extremely slow rate of peptide production,
(2) low peptide production yield,
(3) the peptides that can be produced are limited to those that contain amino acids with comparatively high hydrophobicity,
(4) the amount of enzyme added is extremely large, and
(5) comparatively expensive carboxypeptidase preparations derived from molds, yeasts or plants are required. In the Reaction 4, there is no method known whatsoever that uses an enzyme derived from bacteria or yeasts other than the genus *Saccharomyces,* and there are no known method for producing alanylglutamine and other peptides that are highly hydrophilic. In consideration of this background, there is a need to develop an industrially inexpensive method for producing these peptides.

It is an object of the present invention is to provide a novel enzyme that can produce a peptide easily, inexpensively and at high yield without going through a complex synthesis method. More particularly, it is an object of the present invention to provide a novel enzyme that catalyzes a peptide-producing reaction from a carboxy component and an amine component, a microbe that produces the enzyme, and a method for inexpensively producing peptide using the enzyme or microbe.

As a result of conducting extensive research in consideration of the above object, the inventors of the present invention have found a novel enzyme that efficiently produces peptide from newly discovered bacteria belonging to the genus *Empedobacter* and so forth, and have completed the present invention.

Namely, the present invention is as described below.
[1] An enzyme derived from the genus *Empedobacter* or the genus *Sphingobacterium,* having the ability to produce a peptide from a carboxy component and an amine component.
[2] An enzyme having the ability to produce a peptide from a carboxy component and an amine component that has the ability to produce L-alanyl-L-glutamine at a production rate of 0.03 mM/min or more in a dipeptide-producing reaction under the conditions (i) to (iv):
   (i) the carboxy component is L-alanine methyl ester hydrochloride (100 mM);
   (ii) the amine component is L-glutamine (200 mM);
   (iii) the pH is 9.0; and,
   (iv) the amount of enzyme added is less than 0.61 mg/ml as protein.
[3] The enzyme according to [1] or [2], wherein the carboxy component as a substrate includes both the amino acid ester and the amino acid amide.
[4] The enzyme according to any one of [1] to [3], wherein any of an amino acid, a C-protected amino acid and an amine can be used as a substrate for the amine component.
[5] The enzyme according to any one of [1] to [4], wherein the enzyme has the ability to produce a peptide within a pH range of 6.5 to 10.5.
[6] The enzyme according to any one of [1] to [5], wherein the enzyme has the ability to produce a peptide within a temperature range of 0 to 60°C.
[7] The enzyme according to any one of [1] to [6], wherein the enzyme is not inhibited by a serine enzyme inhibitor, phenylmethylsulfonyl fluoride, but is inhibited by p-nitrophenyl-p'-guanidinobenzoate.
[8] The enzyme according to any one of [1] to [7], wherein the enzyme has a molecular weight as determined by SDS-gel electrophoresis of about 75 kilodaltons, and a molecular weight as determined by gel filtration chromatography of about 150 kilodaltons.
[9] A microbe that produces an enzyme according to any one of [1] to [8].
[10] The microbe according to [9], wherein, the microbe is *Empedobacter brevis* strain FERM BP-8113 (Depositary institution:
   National Institute for Advanced Industrial Science and Technology, International Patent Organism Depositary, Address of deposited institution: Chuo Dai-6, 1-1 Higashi 1-Chome, Tsukuba-shi, Ibaraki-ken, Japan, International deposit transfer date: July 8, 2002) or Sphingobacterium sp. strain FERM BP-8124 (Depositary institution:
   National Institute for Advanced Industrial Science and Technology, International Patent Organism Depository, Address of depositary institution: Chuo Dai-6, 1-1 Higashi 1-Chome, Tsukuba-shi, Ibaraki-ken,, Japan, International deposit transfer date: July 22, 2002).
[11] A method for producing a dipeptide, comprising producing a dipeptide from a carboxy component and an amine component using an enzyme according to any one of [1] to [8] or a substance containing the enzyme.

### BRIEF DESCRIPTION OF THE DRAWINGS

Fig. 1 is a diagram showing an optimum pH of the enzyme of the present invention;
Fig. 2 is a diagram showing an optimum temperature of the enzyme of the present invention; and
Fig. 3 is a diagram showing the time course in L-alanyl-L-glutamine production from L-alanine methyl ester and L-glutamine.

### BEST MODE FOR CARRYING OUT THE INVENTION

Modes for carrying out the present invention are explained in detail in the order of
(1) Microbe Producing the Enzyme of the Present Invention,
(2) Microbe Culturing,
(3) Enzyme Purification,
(4) Enzyme Properties, and
(5) Dipeptide Synthesis Method.

### (1) Microbe Producing the Enzyme of the Present Invention

The enzyme of the present invention may be any enzyme that has the ability to produce a peptide from a carboxy component and an amine component, and there are no particular restriction on organisms that produce such an enzyme. In the present specification, the carboxy component refers to the component that provides a carbonyl site (CO) in the peptide bond (-CONH-), while the amine component refers to the component that provides the amino site (NH) in the peptide bond. In addition, in the present specification, the term "peptide" used alone refers to a polymer having at least one or more peptide bonds unless otherwise indicated specifically. In addition, the term "dipeptide" in the present specification refers to a peptide having one peptide bond.

Examples of microbes that produce the enzyme of the present invention include bacteria belonging to the genus *Empedobacter* and so forth, specific examples of which include *Empedobacter brevis* strain ATCC 14234 (strain FERM P-18545), and *Sphingobacterium sp*. strain FERM BP-8124. *Empedobacter brevis* strain ATCC 14234 (strain FERM P-18545, strain FERM BP-8113) and *Sphingobacterium sp.* strain FERM BP-8124 are microbes that were screened by the inventors of the present invention as a result of searching for microbes that produce a peptide from a carboxy component and an amine component at high yield. Microbes having similar bacteriological properties to those of *Empedobacter brevis* strain ATCC 14234 (strain FERM P-18545) or *Sphingobacterium sp.* strain FERM BP-8124 are also microbes that produce the enzyme of the present invention.

*Empedobacter brevis* strain ATCC 14234 (strain FERM P-18545) was deposited at the International Patent Organism Depository of the National Institute for Advanced Industrial Science and Technology (Central 6, 1-1 Higashi, Tsukuba City, Ibaraki Prefecture, Japan) on October 1, 2001 and assigned the deposit number of FERM P-18545. Control of this organism was subsequently transferred to deposition under the provisions of the Budapest Treaty at the International Patent Organism Depositary of the National Institute for Advanced Industrial Science and Technology on July 8, 2002 and was assigned the deposit number of FERM BP-8113 (indication of microbe: *Empedobacter brevis* strain AJ 13933).

*Sphingobacterium sp.* strain AJ 110003 was deposited at the International Patent Organism Depositary of the National Institute for Advanced Industrial Science and Technology on July 22, 2002, and was assigned the deposit number of FERM BP-8124. It should be noted that strain AJ 110003 was identified to be the aforementioned *Sphingobacterium sp.* by the identification experimentation described below. Strain FERM BP-8124 (Depositary institution: National Institute for Advanced Industrial Science and Technology, International Patent Organism Depositary, Address of depositary institution: Chuo Dai-6, 1-1 Higashi 1-Chome, Tsukuba-shi, Ibaraki-ken,, Japan, International deposit date: July 22, 2002) is a Gram-negative rod (0.7 to 0.8 × 1.5 to 2.0 micrometers (hereinafter, "µm"))that does not form spores and is not motile. Its colonies are round with a completely smooth border, contain low protrusions and have a glossy, light yellow color. The organism grows at 30°C and is catalase positive, oxidase positive and negative for the OF test (glucose), and was identified as a bacterium belonging to the genus *Sphingobacterium* based on these properties. Moreover, from the properties of being negative for nitrate reduction, negative for indole production, negative for production from glucose, arginine dihydrolase negative, urease positive, esculin hydrolysis positive, gelatin hydrolysis negative, β-galactosidase positive, glucose assimilation positive, L-arabinose assimilation negative, D-mannose assimilation positive, D-mannitol assimilation negative, N-acetyl-D-glucosamine assimilation positive, maltose assimilation positive, potassium gluconate assimilation negative, n-capric acid negative, adipic acid assimilation negative, dl-malic acid assimilation negative, sodium citrate assimilation negative, phenyl acetate assimilation negative and cytochrome oxidase positive, the microbe was determined to have properties that are similar to those of *Sphingobacterium multivorum* or *Sphingobacterium spiritivorum.* Moreover, as a result of analyzing homology of the base sequence of 16S rRNA gene, although the highest degree of homology was exhibited with *Sphingobacterium multivorum* (98.8%), there was no strain with which it matched completely. This bacterial strain was therefore identified as *Sphingobacterium sp.*

The enzyme of the present invention can be obtained by isolating and purifying from the cells of the above-mentioned *Empedobacter brevis* or *Sphingobacterium sp.* In addition, the enzyme of the present invention as well as microbes that produce the enzyme can also be obtained by genetic engineering techniques based on the isolated enzyme. Namely, the enzyme and microbe of the present invention can be produced by isolating a DNA that encodes the enzyme of the present invention based on the isolated and purified enzyme followed by expressing the DNA by introducing the DNA into a suitable host. In addition, an enzyme having the ability to produce a peptide from a carboxy component and an amine component can also be obtained from other microbes by producing a probe based on a polynucleotide and so forth that encodes the enzyme of the present invention obtained from *Empedobacter brevis.* Various gene recombination techniques are described in Molecular Cloning, 2nd edition, Cold Spring Harbor Press (1989) and other publications.

### (2) Microbe Culturing

In order to obtain cultured cells of microbes having the enzyme used in the present invention, it suffices that the microbes be cultured and grown in a suitable medium. There are no particular restrictions on the medium used for this purpose provided that it is allows the microbes to grow. This medium may be an ordinary medium containing ordinary carbon sources, nitrogen sources, phosphorus sources, sulfur sources, inorganic ions, and organic nutrient sources as necessary.

For example, any carbon source may be used provided that the microbes can utilize it. Specific examples of the carbon source that can be used include sugars such as glucose, fructose, maltose and amylose, alcohols such as sorbitol, ethanol and glycerol, organic acids such as fumaric acid, citric acid, acetic acid and propionic acid and their salts, hydrocarbons such as paraffin as well as mixtures thereof.

Examples of nitrogen sources that can be used include ammonium salts of inorganic salts such as ammonium sulfate and ammonium chloride, ammonium salts of organic acids such as ammonium fumarate and ammonium citrate, nitrates such as sodium nitrate and potassium nitrate, organic nitrogen compounds such as peptones, yeast extract, meat extract and corn steep liquor as well as mixtures thereof.

In addition, ordinary nutrient sources used in media, such as inorganic salts, trace metal salts and vitamins, can also be suitably mixed and used.

There are no particular restrictions on culturing conditions, and culturing can be carried out, for example, for about 12 to about 48 hours while properly controlling the pH and temperature to a pH range of 5 to 8 and a temperature range of 15 to 40°C, respectively, under aerobic conditions.

### (3) Enzyme Purification

A method for isolating and purifying a peptide-producing enzyme from *Empedobacter brevis* is explained as an example of purifying the enzyme of the present invention. First, a microbial cell extract is prepared from the cells of, for example, *Empedobacter brevis* strain FERM BP-8113 (Depositary institution: National Institute for Advanced Industrial Science and Technology, International Patent Organism Depositary, Address of deposited institution: Central 6, 1-1-1 Higashi, Tsukuba City, Ibaraki Prefecture, Japan, International deposit transfer date: July 8, 2002) by disrupting the cells using a physical method such as ultrasonic crushing or an enzymatic method using a cell wall-dissolving enzyme and removing the insoluble fraction by centrifugal separation and so forth.

A peptide-producing enzyme can then be purified from the cell extract obtained in the above manner by combining ordinary protein purification methods such as anion exchange chromatography, cation exchange chromatography or gel filtration chromatography.

An example of a carrier for use in anion exchange chromatography is Q-Sepharose HP (manufactured by Amersham). The enzyme is recovered in the non-adsorbed fraction under conditions of pH 8.5 when the cell extract containing the enzyme is allowed to pass through a column packed with the carrier.

An example of a carrier for use in cation exchange chromatography is MonoS HR (manufactured by Amersham). After adsorbing the enzyme onto the carrier (in the column) by allowing the cell extract containing the enzyme to pass through a column packed with the carrier and then washing the column, the enzyme is eluted with a buffer solution having a high salt concentration. At that time, the salt concentration may be sequentially increased or gradiently increased. For example, in the case of using MonoS HR, the enzyme adsorbed onto the carrier is eluted at an NaCl concentration of about 0.2 to about 0.5 M.

The enzyme purified in the manner described above can then be further homogeneously purified by gel filtration chromatography and so forth. An example of the carrier for use in gel filtration chromatography is Sephadex 200pg (manufactured by Amersham).

The fraction that contains the present enzyme in the aforementioned purification procedure can be confirmed by assaying the peptide production activity of each fraction according to the method described later.

### (4) Properties of Enzyme of the Present Invention

While the enzyme of the present invention is an enzyme that has the ability to produce a peptide from a carboxy component and an amine component, a preferable mode of the enzyme of the present invention will be explained hereinbelow from the standpoint of its properties.

An enzyme having the abilities described below, for which the dipeptide production rate is used as an indicator, is one preferable mode of the enzyme of the present invention. Namely, a preferable mode of the enzyme of the present invention is an enzyme that has the ability to produce a peptide from a carboxy component and an amine component, and the ability to exhibit a production rate of L-alanyl-L-glutamine of preferably 0.03 mM/min or more, more preferably 0.3 mM/min or more, and particularly preferably 1.0 mM/min or more in the dipeptide-producing reaction under the conditions of (i) to (iv) below. The conditions of the dipeptide-producing reaction are as follows:
(i) The carboxy component is L-alanine methyl ester hydrochloride (100 millimolar (hereinafter, "mM"));
(ii) The amine component is L-glutamine (200 mM);
(iii) The pH is 9.0; and,
(iv) The amount of enzyme added is less than 0.61 mg/ml as protein amount.

The aforementioned amount of enzyme added indicates a final amount of added enzyme that is added to the reaction system, and addition of the enzyme of 0.01 mg/ml or more, and preferably 0.02 mg/ml or more, as protein amount is desirable. The term "protein amount" refers to the value indicated with the Coomassie brilliant blue colorimetric method using protein assay CBB solution (manufactured by Nakarai) and bovine serum albumin for the standard substance.

The aforementioned production rate far exceeds the conventional production rate for peptide production using an enzyme, and the enzyme of the present invention has the ability to catalyze peptide production at an extremely high rate.

As a specific example of a procedure for assaying enzyme activity, enzyme activity can be assayed by allowing the enzyme to react in a borate buffer solution containing an amino acid ester and an amine as substrates followed by quantifying the resulting peptide. As a more specific example, the enzyme is allowed to react for several minutes at 25°C using a 100 mM borate buffer solution (pH 9.0) containing 100 mM L-alanine methyl ester and 200 mM L-glutamine.

The enzyme activity unit used in the present invention is defined such that 1 unit (U) is the amount of enzyme that produces 1 micromole of peptide in 1 minute under the condition of reacting at 25°C using 100 mM borate buffer solution (pH 9.0) containing 100 mM L-alanine methyl ester and 200 mM L-glutamine.

In addition, an example of a preferable mode of the enzyme of the present invention is an enzyme having the property by which any of amino acid ester and amino acid amide can be used as a substrate for the carboxy component. The wordings "both an amino acid ester and an amino acid amide can be used as a substrate" mean that at least one type or more of amino acid ester and at least one type or more of amino acid amide can be used as a substrate. In addition, one preferable mode of the enzyme of the present invention is an enzyme that has the property by which any of an amino acid, a C-protected amino acid and an amine can be used as a substrate for the amine component. The wordings "an amino acid, a C-protected amino acid, and an amine can be used as a substrate" mean that at least one type or more of amino acid, at least one type or more of C-protected amino acid and at least one type or more of amine can be used as a substrate. As a result of having a wide range of substrate specificity with respect to the carboxy component or the amino component, the enzyme of the present invention is preferable in the sense that a wide range of raw materials can be selected, which in turn is favorable in terms of cost and production equipment in the case of industrial production.

Specific examples of carboxy components include L-amino acid esters, D-amino acid esters, L-amino acid amides and D-amino acid amides. In addition, amino acid esters include not only amino acid esters corresponding to naturally-occurring amino acids, but also amino acid esters corresponding to non-naturally-occurring amino acids or their derivatives. Further, examples of amino acid esters include α-amino acid esters as well as β-, γ-, and ω-amino acid esters and the like, which have different amino group bonding sites. Typical examples of amino acid esters include methyl esters, ethyl esters, n-propyl esters, iso-propyl esters, n-butyl esters, iso-butyl esters and tert-butyl esters of amino acids, etc.

Specific examples of the amine components include L-amino acids, C-protected L-amino acids, D-amino acids, C-protected D-amino acids and amines. In addition, examples of the amines include not only naturally-occurring amines, but also non-naturally-occurring amines or their derivatives. In addition, examples of the amino acids include not only naturally-occurring amino acids, but also non-naturally-occurring amino acids or their derivatives. These include α-amino acids as well as β-, γ- or ω-amino acids, which have different amino group bonding sites.

In addition, in a different aspect, one preferable mode of the enzyme of the present invention is an enzyme in which the pH range over which the peptide-producing reaction can be catalyzed is 6.5 to 10.5. The ability of the enzyme of the present invention to catalyze this reaction over a wide pH range is preferable in that it allows flexible accommodation of industrial production that could be subject to the occurrence of various restrictions. However, in the actual production of peptide, it is preferable to use the enzyme by further adjusting to an optimum pH corresponding to the acquired enzyme so as to maximize the catalytic performance of the enzyme.

Moreover, an example of another different aspect of a preferable mode of the enzyme of the present invention is an enzyme for which the temperature range over which the enzyme is capable of catalyzing the peptide-producing reaction is within the range of 0 to 60°C. Since the enzyme of the present invention is able to catalyze the reaction over a wide temperature range, it is preferable in that it allows flexible accommodation of industrial production that could be subject to the occurrence of various restrictions. However, in the actual production of peptides, it is preferable to use the enzyme by further adjusting to an optimum temperature corresponding to the acquired enzyme so as to maximize the catalytic performance of the enzyme.

### (5) Dipeptide Synthesis Method

The method for producing a dipeptide according to the present invention comprises synthesizing a dipeptide by allowing an enzyme having the ability to produce a peptide from a carboxy component and an amine component or a substance that contains that enzyme to act on the carboxy component and the amine component.

For the method for allowing the enzyme used in the present invention or enzyme-containing substance to act on the carboxy component and the amine component, it suffices that the enzyme or enzyme-containing substance, carboxy component and amine component be mixed. More specifically, a method may be used in which an enzyme or enzyme-containing substance is added to a solution containing the carboxy component and the amine component and allowed to react, or in the case of using a microbe that produces the enzyme, a method may be used in which the microbe that produces the enzyme is cultured, the enzyme present in the microbe or microbial culture broth is purified and accumulated, and the carboxy component and amine component are then added to the culture broth. The synthesized dipeptide can then be collected by established methods and purified as necessary.

The term "enzyme-containing substance" refers to that which contains the enzyme, and examples of specific forms thereof include a culture of microbes that produce the enzyme, microbial cells isolated from the culture, and a treated microbial cell product. A culture of microbes refers to that which is obtained by culturing microbes, and more specifically, to a mixture of microbial cells, medium used for culturing the microbes, and substances produced by the cultured microbes and the like. In addition, the microbial cells may be washed and used in the form of washed microbial cells. Further, a treated microbial cell product includes the crushed, lysed or freeze-dried microbial cells, and also includes a crude enzyme recovered by processing microbial cells and so forth as well as a purified enzyme obtained by purification of the crude enzyme. A partially purified enzyme obtained by various types of purification methods may be used for the purified enzyme, or immobilized enzyme may be used that has been immobilized by covalent bonding, adsorption or entrapment methods. In addition, since some microbes are partially lysed during culturing depending on the microbes used, the culture supernatant may also be used as the enzyme-containing substance in such cases.

In addition, not only wild strains but also genetic recombinant strains may be used for the microbes that contain the enzyme. The microbes are not limited to intact cells, but rather acetone-treated microbial cells, freeze-dried microbial cells or other treated microbial cells may also be used. Immobilized microbial cells immobilized by covalent bonding, adsorption, entrapment or other methods, as well as treated immobilized microbial cells, may also be used.

It should be noted that in the case of using cultures, cultured microbial cells or a treated microbial cell product, there are many cases in which an enzyme exists that does not participate in production of peptides but decomposes the produced peptides, and in such cases, it is rather preferable to add a metal protease inhibitor like ethylenediaminetetraacetic acid (EDTA) depending on the cases. The addition amount is within the range of 0.1 mM to 300 mM, and preferably 1 mM to 100 mM.

The amount of enzyme or enzyme-containing substance used should be an amount at which the target effect is demonstrated (hereinafter, "effective amount"). Although this effective amount can be easily determined through simple, preliminary experimentation by a person with ordinary skill in the art, in the case of using an enzyme, for example, the use amount thereof is about 0.01 to 100 units (U), while in the case of using washed microbial cells, the use amount thereof is about 1 to 500 g/L.

Any carboxy component may be used provided that it is capable of producing a peptide by condensation with the other substrate in the form of the amine component. Examples of the carboxy components include L-amino acid esters, D-amino acid esters, L-amino acid amides and D-amino acid amides. Examples of the amino acid esters include not only amino acid esters corresponding to naturally-occurring amino acids, but also amino acid esters corresponding to non-naturally-occurring amino acids or their derivatives. In addition, examples of the amino acid esters include α-amino acid esters as well as β-, γ- and ω-amino acid esters and the like, which having different amino group bonding sites. Typical examples of the amino acid esters include methyl esters, ethyl esters, n-propyl esters, iso-propyl esters, n-butyl esters, iso-butyl esters and tert-butyl esters of amino acids.

Any amine component may be used provided that it is capable of producing peptide by condensation with the other substrate in the form of the carboxy component. Examples of the amine components include L-amino acids, C-protected L-amino acids, D-amino acids, C-protected D-amino acids and amines. In addition, examples of the amines include not only naturally-occurring amines, but also non-naturally-occurring amines or their derivatives. Examples of the amino acids include not only naturally-occurring amino acids, but also non-naturally-occurring amino acids or their derivatives. These include α-amino acids as well as β-, γ- or ω-amino acids, which have different amino group bonding sites.

Although the concentrations of the carboxy component and amine component serving as starting materials are 1 mM to 10 M, and preferably 0.05 mole (hereinafter, "M") to 2 M, respectively, there are cases in which it is preferable to add the amine component in an amount equal to or greater than that of the carboxy component. In addition, in the case of substrates that inhibit the reaction at high concentrations, these can be adjusted to a concentration that does not result in inhibition and successively added during the reaction.

The reaction temperature that allows production of a peptide is 0 to 60°C, and preferably 5 to 40°C. The reaction pH that allows production of a peptide is 6.5 to 10.5, and preferably 7.0 to 10.0.

### Examples

Although the following provides a more detailed explanation of the present invention through its examples, the present invention is not limited to these examples. In addition to confirmation by ninhydrin coloring of thin-film chromatograms (qualitative), quantitative determinations were made by the following high-performance liquid chromatography in order to assay products. Column: InertsiL ODS-2 (manufactured by GL Science, Inc.), eluate: aqueous phosphate solution containing 5.0 mM sodium 1-octanesulfonate (pH 2.1) : methanol = 100 : 15 to 50, flow rate: 1.0 mL/min, detection: 210 nanometers (hereinafter, "nm").

### (Example 1) Microbe Culturing (Empedobacter brevis Strain FERM BP-8113)

A 50 mL medium (pH 6.2) containing 5 grams (hereinafter, "g") of glucose, 5 g of ammonium sulfate, 1 g of monopotassium phosphate, 3 g of dipotassium phosphate, 0.5 g of magnesium sulfate, 10 g of yeast extract and 10 g of peptone in 1 liter (hereinafter, "L") was transferred to a 500 mL Sakaguchi flask and sterilized at 115°C for 15 minutes. This medium was then inoculated with one loopful of the culture broth of *Empedobacter brevis* strain FERM BP-8113 (Depositary institution: National Institute for Advanced Industrial Science and Technology, International Patent Organism Depositary, Address of depositary institution: Chuo Dai-6, 1-1 Higashi 1-Chome, Tsukuba-shi, lbaraki-ken, Japan, International deposit transfer date: July 8, 2002) that had been cultured at 30°C for 16 hours in the same medium, followed by shake culturing at 30°C for 16 hours and 120 strokes/min.

### (Example 2) Production of Peptide Using Microbial Cells

Microbial cells were collected by centrifuging (10,000 rounds per minute (hereinafter, "rpm"), 15 minutes) the culture broth obtained in Example 1 followed by suspending to a concentration of 100 g/L in 100 mM borate buffer (pH 9.0) containing 10 mM EDTA. After respectively adding 1 mL of this suspension to 1 mL of 100 mM borate buffer (pH 9.0) containing 10 mM EDTA, 200 mM of the following carboxy component and 400 mM of the following amino acids to bring to a final volume of 2 mL, the reaction was carried out at 18°C for 2 hours. The peptides that were produced as a result of this reaction are shown in Table 1.

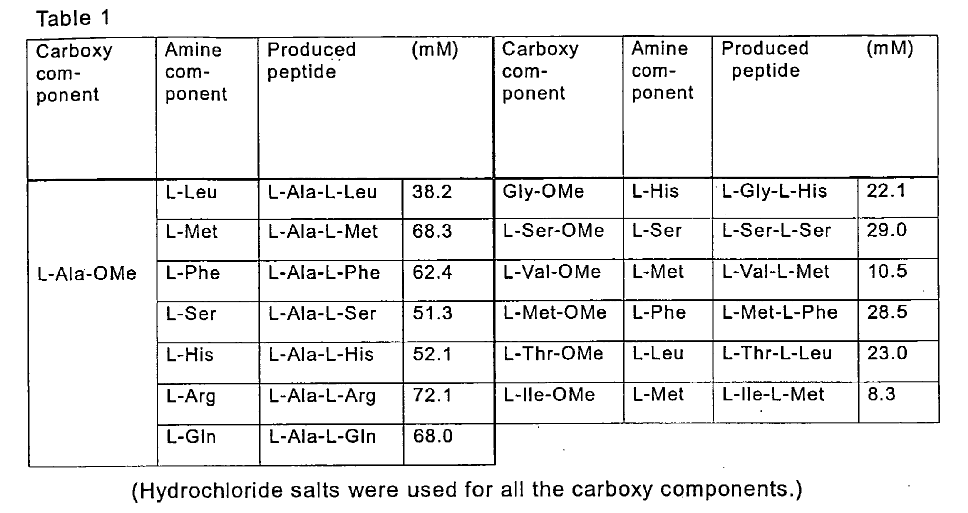

### (Example 3) Enzyme Purification

The procedure after centrifugal separation was carried out either on ice or at 4°C. *Empedobacter brevis* strain FERM BP-8113 (Depositary institution: National Institute for Advanced Industrial Science and Technology, International Patent Organism Depositary, Address of depositary institution: Chuo Dai-6, 1-1 Higashi 1-Chome, Tsukuba-shi, Ibaraki-ken,, Japan, International deposit transfer date: July 8, 2002) was cultured in the same manner in as Example 1, and the microbial cells were collected by centrifugal separation (10,000 rpm, 15 minutes). After washing 16 g of microbial cells with 50 mM Tris-HCl buffer (pH 8.0), they were suspended in 40 milliliters (hereinafter, "ml" or "mL") of the same buffer and subjected to ultrasonic crushing treatment for 45 minutes at 195 watts. This ultrasonic crushing liquid was then centrifuged (10,000 rpm, 30 minutes) to remove the crushed cell fragments and obtain an ultrasonic crushing liquid supernatant. This ultrasonic crushing liquid supernatant was dialyzed overnight against 50 mM Tris-HCl buffer (pH 8.0) followed by removal of the insoluble fraction by ultracentrifugation (50,000 rpm, 30 minutes) to obtain a soluble fraction in the form of the supernatant liquid. The resulting soluble fraction was applied to a Q-Sepharose HP column (manufactured by Amersham) pre-equilibrated with Tris-HCl buffer (pH 8.0), and the active fraction was collected from the non-adsorbed fraction. This active fraction was dialyzed overnight against 50 mM acetate buffer (pH 4.5) followed by removal of the insoluble fraction by centrifugal separation (10,000 rpm, 30 minutes) to obtain a dialyzed fraction in the form of the supernatant liquid. This dialyzed fraction was then applied to a Mono S column (manufactured by Amersham) pre-equilibrated with 50 mM acetate buffer (pH 4.5) to elute enzyme at a linear concentration gradient of the same buffer containing 0 to 1 M NaCl. The fraction that had the lowest level of contaminating protein among the active fractions was applied to a Superdex 200pg column (manufactured by Amersham) pre-equilibrated with 50 mM acetate buffer (pH 4.5) containing 1 M NaCl, and gel filtration was performed by allowing the same buffer (pH 4.5) containing 1 M NaCl to flow through the column to obtain an active fraction solution. As a result of performing these procedures, the peptide-producing enzyme used in the present invention was confirmed to have been uniformly purified based on the experimental results of electrophoresis. The enzyme recovery rate in the aforementioned purification process was 12.2% and the degree of purification was 707 times.

### (Example 4) Measurement of Enzyme Molecular Weight

### (SDS-Gel Electrophoresis)

A 0.3 microgram (µg) of the purified enzyme fraction obtained by the method of Example 3 was applied to polyacrylamide electrophoresis. 0.3% (w/v) Tris, 1.44% (w/v) glycine and 0.1 % (w/v) sodium laurylsulfate were used for the electrophoresis buffer solution, a gel having a concentration gradient of a gel concentration of 10 to 20% (Multigel 10 to 20, manufactured by Daiichi Pure Chemicals) was used for the polyacrylamide gel, and Pharmacia molecular weight markers were used for the molecular weight markers. Following completion of electrophoresis, the gel was stained with Coomassie brilliant blue R-250, and a uniform band was detected at the location of a molecular weight of about 75 kilodalton (hereinafter, "kDa").

### (Gel filtration)

The purified enzyme fraction obtained by the method of Example 3 was applied to a Superdex 200pg column (manufactured by Amersham) pre-equilibrated with 50 mM acetate buffer (pH 4.5) containing 1 M NaCl, and gel filtration was carried out by allowing the same buffer (pH 4.5) containing 1 M NaCl to flow through the column to measure the molecular weight. Pharmacia molecular weight markers were used as standard proteins having known molecular weights to prepare a calibration curve. As a result, the molecular weight of the enzyme was about 150 kDa.

Based on the results of SDS-gel electrophoresis and gel filtration, the enzyme was suggested to be a homodimer having a molecular weight of about 75 kDa.

### (Example 5) Enzyme Optimum pH

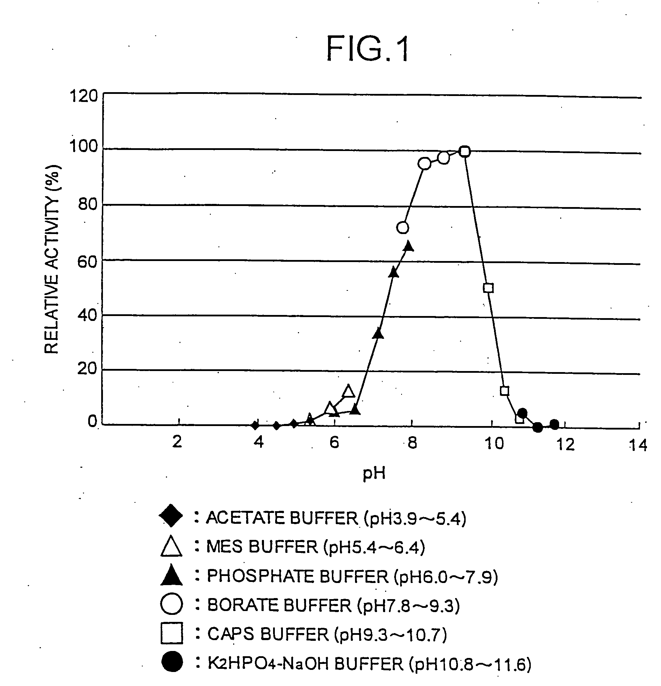

The effects of pH were examined in the reaction in which L-alanyl-L-glutamine is produced from L-alanine methyl ester hydrochloride and L-glutamine. Acetate buffer (pH 3.9 to 5.4), MES buffer (pH 5.4 to 6.4), phosphate buffer (pH 6.0 to 7.9), borate buffer (pH 7.8 to 9.3), CAPS buffer (pH 9.3 to 10.7) and K₂HPO₄-NaOH buffer (pH 10.8 to 11.6) were used as buffers. 1 microliter (µl) of the Mono S fraction enzyme obtained in Example 3 (about 180 U/ml) was added to 100 µl of each buffer (100 mM) containing 100 mM L-alanine methyl ester, 200 mM L-glutamine and 10 mM EDTA and allowed to react at 18°C for 5 minutes to measure the effects of pH on the reaction. The results based on assigning a value of 100% to the case of using borate buffer (pH 9.3) are shown in Fig. 1. As a result, the optimum enzyme pH was 8 to 9.5.

### (Example 6) Enzyme Optimum Temperature

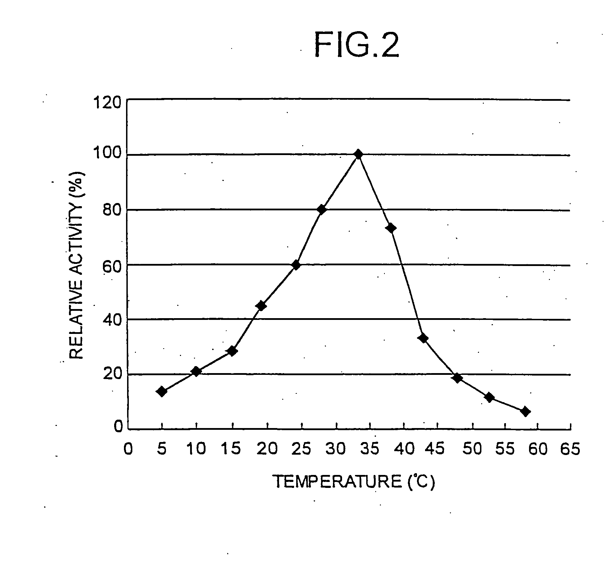

The effects of temperature were examined on the reaction in which L-alanyl-L-glutamine is produced from L-alanine methyl ester hydrochloride and L-glutamine. A 1 µl aliquot of the same enzyme fraction used in Example 5 was added to 100 µl of 100 mM borate buffer (pH 9.0) containing 100 mM L-alanine methyl ester, 200 mM L-glutamine and 10 mM EDTA and allowed to react for 5 minutes at each temperature to measure the effects of temperature on the reaction. The results based on assigning a value of 100% to the activity at 34°C are shown in Fig. 2. As a result, the optimum enzyme temperature was 30 to 40°C.

### (Example 7) Enzyme Inhibitors

The effects of inhibitors on the production of L-alanyl-L-glutamine were examined using L-alanine methyl ester hydrochloride and L-glutamine as substrates. A 2 µl aliquot of the same enzyme fraction used in Example 5 was added to 50 µl of 100 mM borate buffer (pH 9.0) containing each of the enzyme inhibitors shown in Table 2 at 10 mM, and allowed to react at 25°C for 5 minutes. Note that, o-phenanthroline, phenylmethylsulfonyl fluoride and p-nitrophenyl-p'-guanidinobenzoate were dissolved in methanol to a concentration of 50 mM before use. The enzyme activity under each condition was indicated as the relative activity in the case of assigning a value of 100 to the production of L-alanyl-L-glutamine in the absence of enzyme inhibitor. Those results are shown in Table 2. As a result, among the serine enzyme inhibitors tested, the enzyme was not inhibited by phenylmethylsulfonyl fluoride, but it was inhibited by p-nitrophenyl-p'-guanidinobenzoate.

**Table 2**

| Enzyme inhibitor | | Relative activity of L-Ala-L-Gln production (%) |
|---|---|---|
| None | | 100 |
| Metal enzyme inhibitor | EDTA | 96 |
| | o-Phenanthroline | 96 |
| SH enzyme inhibitor | N-Ethyl maleimide | 110 |
| | Monoiodoacetate | 101 |
| Serine enzyme inhibitor | Phenylmethylsulfonyl fluoride | 115 |
| | 4-(2-Aminoethyl)benzene sulfonyl fluoride | 75 |
| | p-Nitrophenyl-p'-guanidino benzoate | 0.1 |

### (Example 8) Production of L-Alanyl-L-Glutamine from L-Alanine Methyl Ester and L-Glutamine

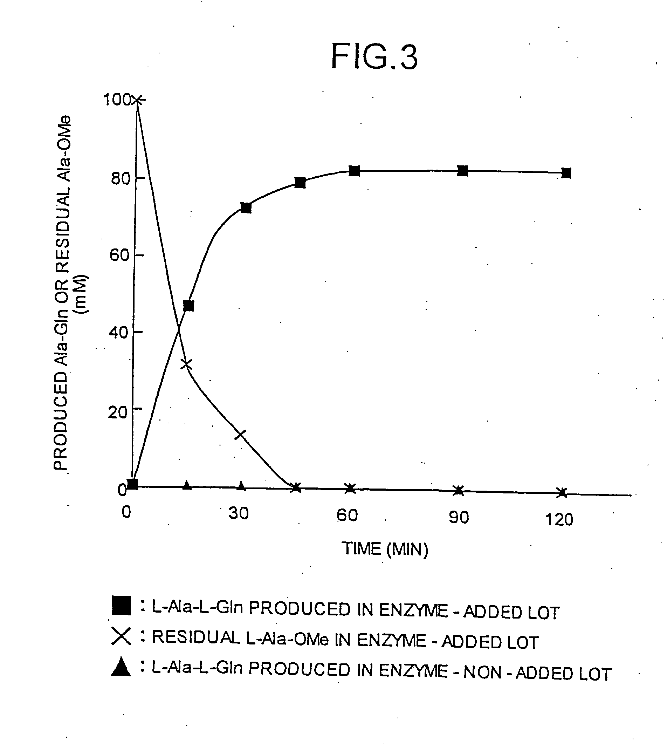

A 3 µl aliquot of the same enzyme fraction as used in Example 5 was added to 100 µl of 100 mM borate buffer (pH 9.0) containing 100 mM L-alanine methyl ester hydrochloride, 200 mM L-glutamine and 10 mM EDTA, and allowed to react at 18°C. As a result, as shown in Fig. 3, 83 mM L-alanyl-L-glutamine (L-Ala-L-Gln) was produced in the case of an enzyme-added lot, and the concentration of by-product L-Ala-L-Ala-L-Gln was 1.3 mM. On the other hand, there was hardly any production of L-Ala-L-Gln observed in an enzyme-non-added lot, and the enzyme concentration was only about 0.07 mM after reacting for 120 minutes.

### (Example 9) Effects of L-Glutamine Concentration on Production of L-Alanyl-L-Glutamine

A 1 µl aliquot of the same enzyme fraction as used in Example 5 was added to 100 µl of 100 mM borate buffer (pH 9.0) containing 100 mM L-alanine methyl ester hydrochloride, L-glutamine at the concentrations shown in Table 3 and 10 mM EDTA, and allowed to react at 18°C for 2 hours. Those results are shown in Table 3.

**Table 3**

| L-Alanine methyl ester hydrochloride (mM) | L-Glutamine (mM) | L-Ala-L-Gln (mM) |
|---|---|---|
| 100 | 100 | 68.2 |
| | 110 | 72.1 |
| | 120 | 73.3 |
| | 130 | 75.1 |
| | 150 | 75.5 |
| | 200 | 82.0 |

### (Example 10) Enzyme Substrate Specificity (1)

Ester specificity was examined in the case of using L-amino acid ester for the carboxy component. A 2 µl aliquot of the same enzyme fraction as used in Example 5 was added to 100 µl of 100 mM borate buffer (pH 9.0) containing the carboxy components indicated in Table 4 at 100 mM, 200 mM L-glutamine and 10 mM EDTA, and allowed to react at 25°C for 2 hours. The amounts of L-Ala-L-Gln produced in this reaction are shown in Table 4 (HCl represents hydrochloride in Table 4).

**Table 4**

| Carboxy component | L-Ala-L-Gln produced (mM) |
|---|---|
| L-Alanine methyl ester·HCl | 84.3 |
| L-Alanine ethyl ester·HCl | 91.5 |
| L-Alanine isopropyl ester·HCl | 78.9 |
| L-Alanine-t-butyl ester·HCl | 7.5 |

### (Example 11) Enzyme Substrate Specificity (2)

Peptide production was examined in the case of using L-alanine methyl ester for the carboxy component and using various L-amino acids for the amine component. A 2 µl aliquot of the same enzyme fraction as used in Example 5 was added to 100 µl of 100 mM borate buffer (pH 9.0) containing 100 mM L-alanine methyl ester hydrochloride, the L-amino acids shown in Table 5 at 150 mM and 10 mM EDTA, and allowed to react at 25°C for 3 hours. The amounts of each of the peptides produced in this reaction are shown in Table 5. (The "+" mark indicates those peptides for which production was confirmed but which were unable to be quantified due to the absence of a standard, while "tr" indicates a trace amount.)

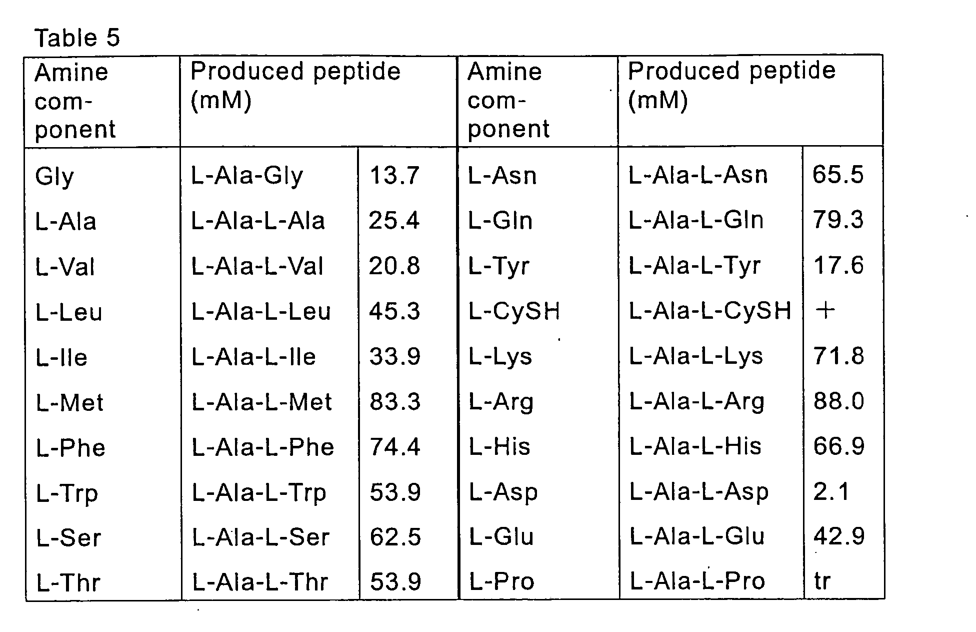

### (Example 12) Enzyme Substrate Specificity (3)

Peptide production was examined in the case of using various types of L-amino acid methyl esters for the carboxy component and using L-glutamine for the amine component. A 2 µl aliquot of the same enzyme fraction as used in Example 5 was added to 100 µl of 100 mM borate buffer (pH 9.0) containing the L-amino acid methyl ester hydrochlorides (AA-OMe·HCl) shown in Table 6 at 100 mM, 150 mM L-glutamine and 10 mM EDTA, and allowed to react at 25°C for 3 hours. The amounts of each of the peptides produced in this reaction are shown in Table 6. (The "+" mark indicates those peptides for which production was confirmed but which were unable to be quantified due to the absence of a standard, while "tr" indicates a trace amount.) Furthermore, Tween-80 was added to the reaction system to a final concentration of 0.1% in the case of using L-Trp-OMe and L-Tyr-OMe.

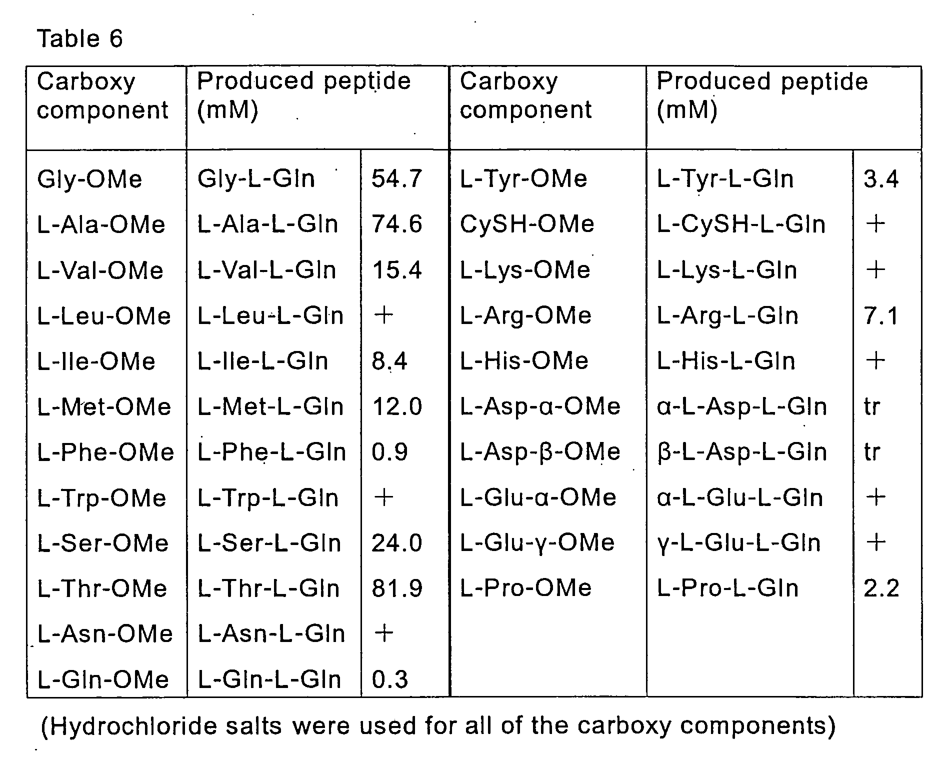

### (Example 13) Enzyme Substrate Specificity (4)

Peptide production was examined in the case of using various L-amino acid methyl esters for the carboxy component and various L-amino acids for the amine component. A 2 µl aliquot of the same enzyme fraction as used in Example 5 was added to 100 µl of 100 mM borate buffer (pH 9.0) containing the L-amino acid methyl ester hydrochlorides (AA-OMe·HCl) shown in Table 7 at 100 mM, the L-amino acids shown in Table 7 at 150 mM and 10 mM EDTA, and allowed to react at 25°C for 3 hours. The amounts produced of each of the peptides produced in this reaction are shown in Table 7. (The "tr" indicates a trace amount.) Furthermore, Tween-80 was added to the reaction system to a final concentration of 0.1 % in the case of using L-Trp-OMe. (The "+" mark indicates those peptides for which production was confirmed but which were unable to be quantified due to the absence of a standard.)

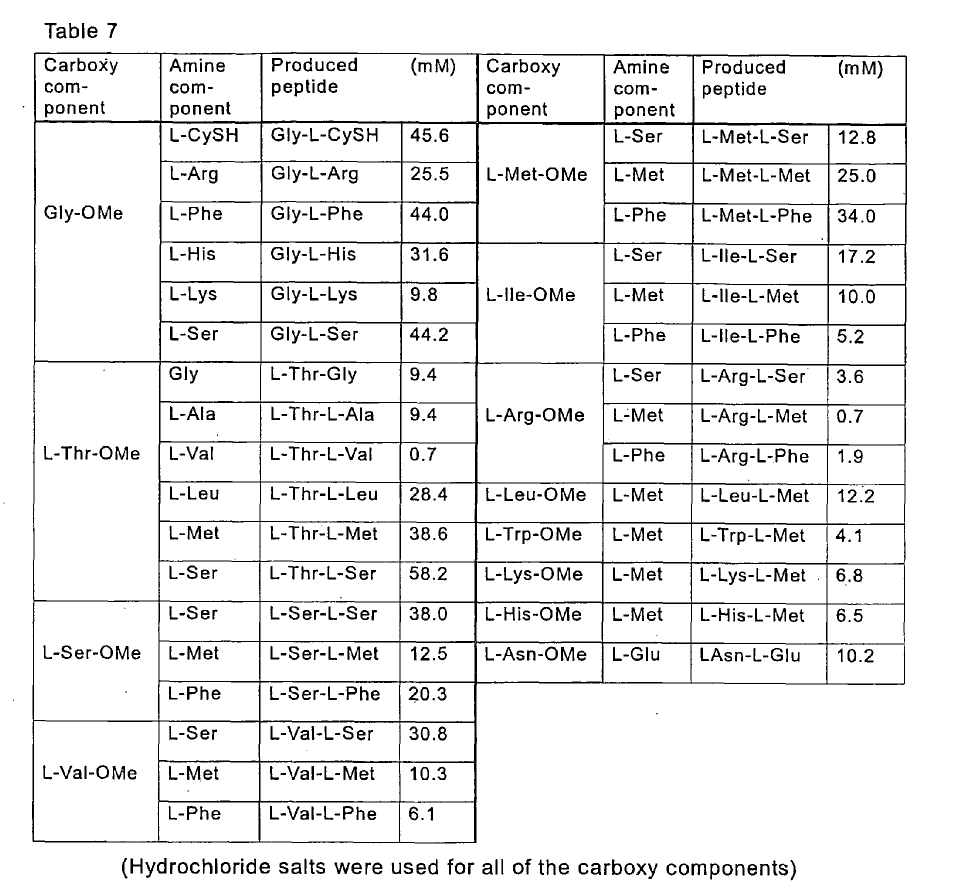

### (Example 14) Enzyme Substrate Specificity (5)

Peptide production was examined in the case of using the L or D forms of various amino acid methyl esters for the carboxy component, and the L or D forms of various amino acids for the amine component. 2 µl of the same enzyme fraction as used in Example 5 was added to 100 µl of 100 mM borate buffer (pH 9.0) containing the various amino acid methyl ester hydrochlorides (AA-OMe·HCl) shown in Table 8 at 100 mM, the various amino acids shown in Table 8 at 150 mM and 10 mM EDTA, and allowed to react at 25°C for 3 hours. The amounts of each of the peptides produced in this reaction are shown in Table 8. (The "tr" indicates a trace amount.)

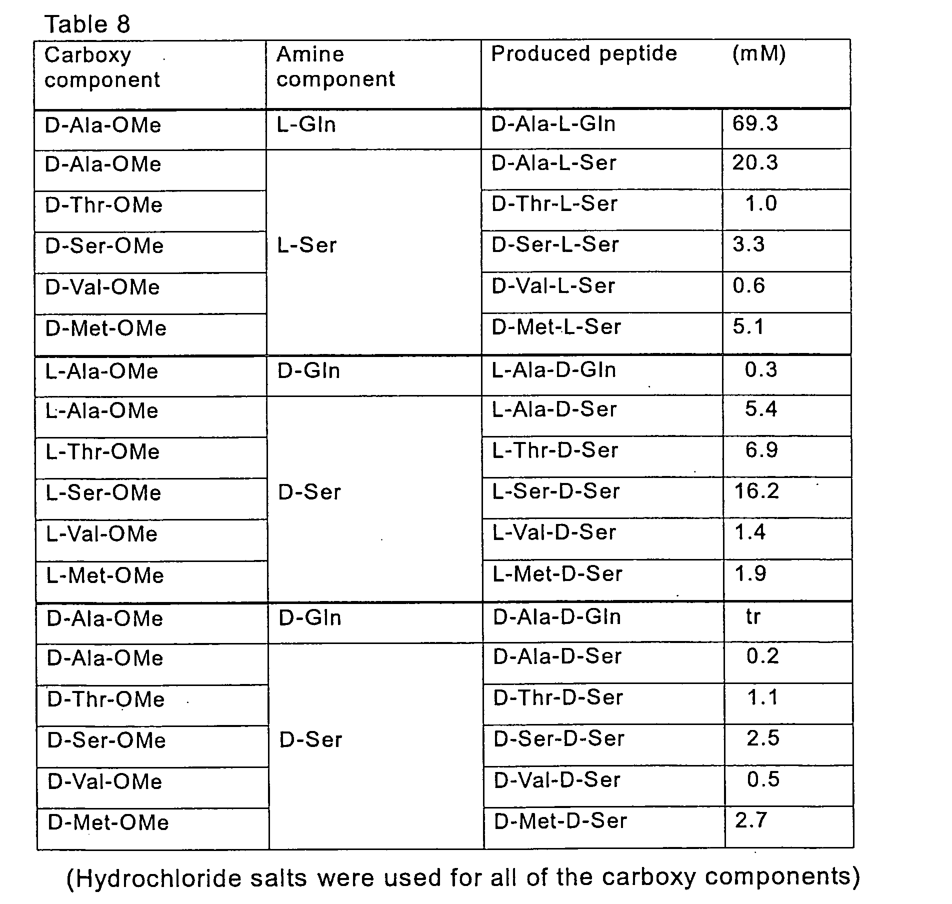

### (Example 15) Enzyme Substrate Specificity (6)

Peptide production was examined using various L-amino acid amides for the carboxy component, and various L-amino acids for the amine component. A 2 µl aliquot of the same enzyme fraction as that used in Example 5 was added to 100 µl of 100 mM borate buffer (pH 9.0) containing the L-amino acid amide hydrochloride salts (AA-NH₂·HCl) shown in Table 9 at 100 mM, the L-amino acids shown in Table 9 at 150 mM and 10 mM EDTA, and allowed to react at 25°C for 3 hours. The amounts of each of the peptides produced in this reaction are shown in Table 9.

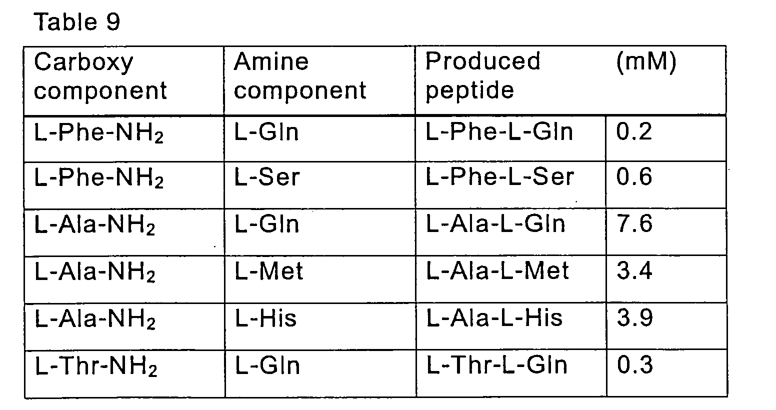

### (Example 16) Enzyme Substrate Specificity (7)

Peptide production was examined in the case of using various L-alanine methyl esters for the carboxy component and C-protected L-amino acids for the amine component. A 2 µl aliquot of the same enzyme fraction as used in Example 5 was added to 100 µl of 100 mM borate buffer (pH 9.0) containing the L-amino acid methyl ester (AA-OMe·HCl) shown in Table 10 at 100 mM, the L-amino acid amides shown in Table 10 at 150 mM and 10 mM EDTA, and allowed to react at 25°C for 3 hours. The amounts of each of the peptides produced in this reaction are shown in Table 10.

**Table 10**

| Carboxy component | Amine component | Produced peptide | (mM) |
|---|---|---|---|
| L-Ala-OMe | Gly-NH₂ | L-Ala-Gly-NH₂ | 7.4 |
| | L-Ala-NH₂ | L-Ala-L-Ala-NH₂ | 8.3 |
| | L-Phe-NH₂ | L-Ala-L-Phe-NH₂ | 12.2 |

### (Example 17) Enzyme Substrate Specificity (8)

Peptide production was examined in the case of using various amino acid methyl esters for the carboxy component and methylamine for the amine component. A 2 µl aliquot of the same enzyme fraction as used in Example 5 was added to 100 µl of 100 mM borate buffer (pH 9.0) containing the amino acid methyl ester (AA-OMe·HCl) shown in Table 11 at 100 mM, the methylamine shown in Table 11 at 150 mM and 10 mM EDTA, and allowed to react at 25°C for 3 hours. The amounts of each of the peptides produced in this reaction are shown in Table 11.

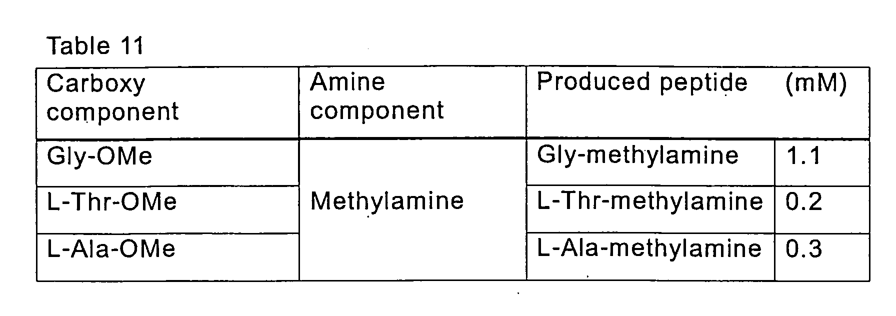

### (Example 18) Enzyme Substrate Specificity (9)

Peptide production was examined in the case of using β-amino acid ester for the carboxy component or β-amino acid for the amine component. A 2 µl aliquot of the same enzyme fraction as used in Example 5 was added to 100 µl of 100 mM borate buffer (pH 9.0) containing the carboxy components shown in Table 12 at 100 mM, the amine components shown in Table 12 at 150 mM and 10 mM EDTA, and allowed to react at 25°C for 3 hours. The amounts of each of the peptides produced in this reaction are shown in Table 12. (The "tr" indicates a trace amount.)

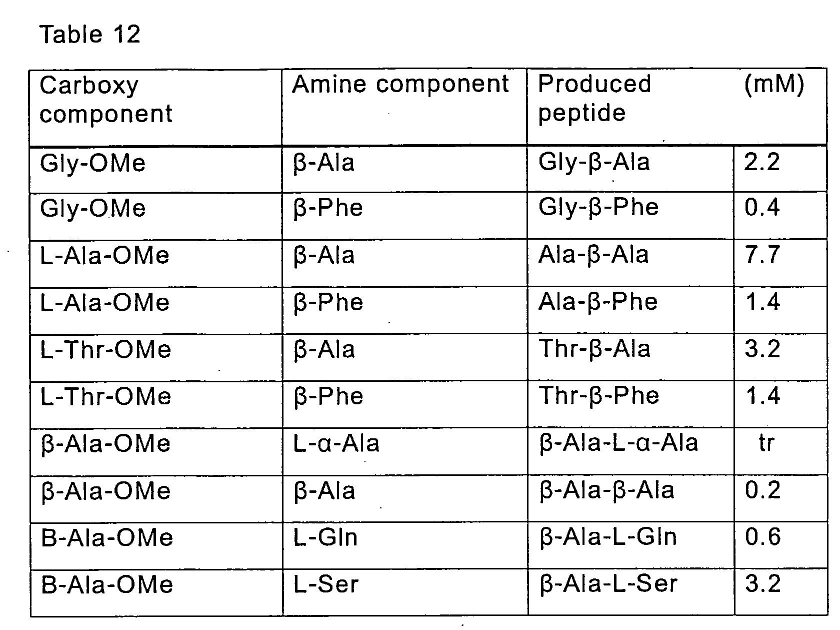

### (Example 19) Enzyme Substrate Specificity (10)

Oligopeptide production was examined in the case of using L-amino acid ester for the carboxy component and peptide for the amine component. A 2 µl aliquot of the same enzyme fraction as used in Example 5 was added to 100 µl of 100 mM borate buffer (pH 9.0) containing the carboxy components shown in Table 13 at 100 mM, the amine components shown in Table 13 at 150 mM and 10 mM EDTA, and allowed to react at 25°C for 3 hours. The amounts of each of the peptides produced in this reaction are shown in Table 13. As a result, it was clearly demonstrated that the present enzyme can produce not only dipeptide, but also long-chain peptides by using a peptide for the amine component.

As has been indicated in the aforementioned Examples 9 to 19, the present enzyme obtained from *Empedobacter brevis* strain FERM P BP-8113 (Depositary institution: National Institute for Advanced Industrial Science and Technology, International Patent Organism Depositary, Address of depositary institution: Chuo Dai-6, 1-1 Higashi 1-Chome, Tsukuba-shi, Ibaraki-ken, Japan, International deposit transfer date: July 8, 2002) was determined to have an extremely broad substrate specificity.

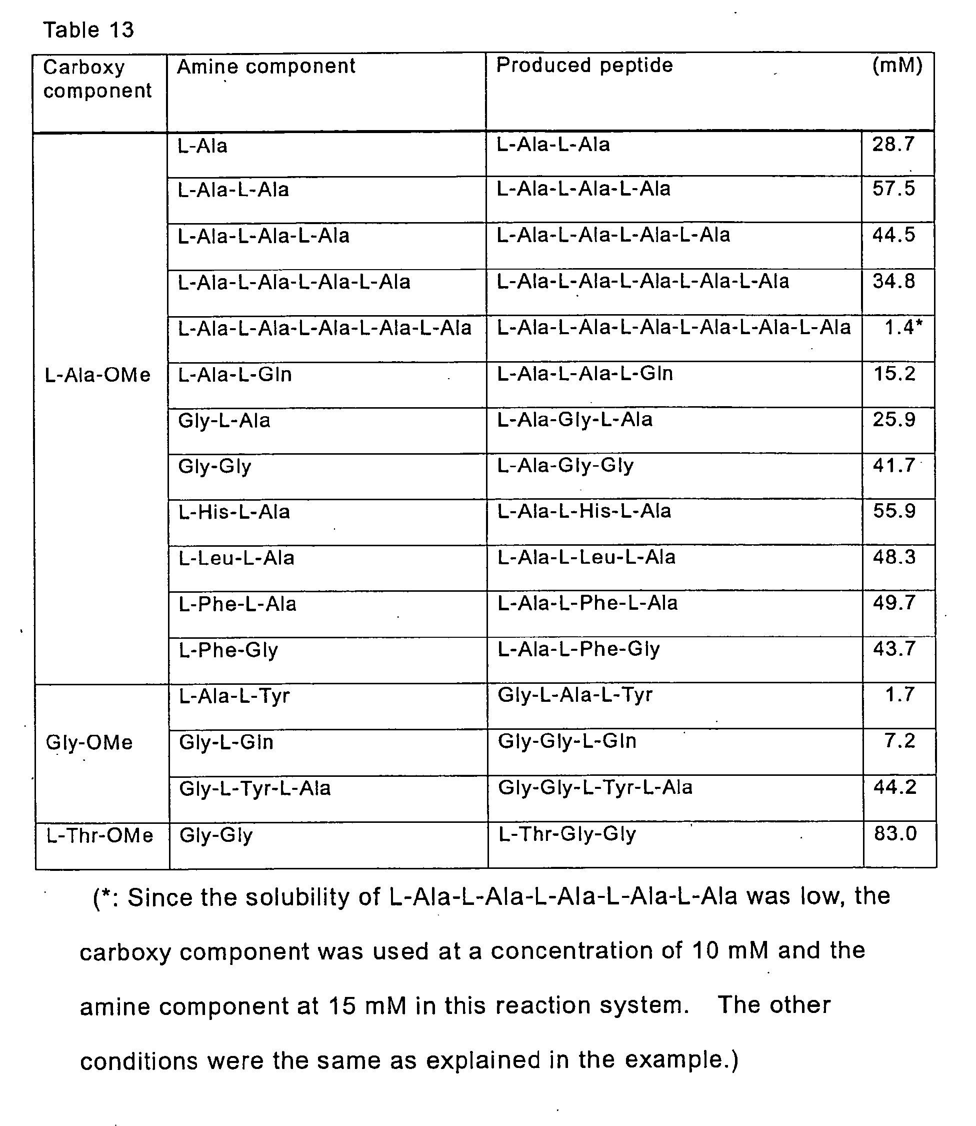

### (Example 20) Comparison of Ability to Catalyze Peptide Production with Known Enzymes

The peptide formation ability of the present enzyme was compared with that of known enzymes. Carboxypeptidase Y described in EP 278787A1 and the thiol endopeptidases (ficin, papain, bromelain and chymopapain) described in EP 359399B1 were used for the known enzymes, and they were used in the form of purified enzymes manufactured by Sigma. The enzyme uniformly purified in Example 3 was used for the source of the enzyme of the present invention. These enzymes were added to a reaction system in the amounts shown in Table 14 as protein. The reaction was carried out by adding enzyme to 100 µl of borate buffer (pH 9.0) containing 100 mM L-alanine methyl ester and 200 mM L-glutamine and allowing to react at 25°C. Furthermore, enzyme dissolved in 10 mM acetate buffer (pH 5.0) containing 1 mM EDTA was used for the carboxypeptidase, while enzyme dissolved in 10 mM acetate buffer (pH 5.0) containing 2 mM EDTA, 0.1 M KCI and 5 mM dithiothreitol was used for the thiol endopeptidase. The ratios of the production rates of L-alanyl-L-glutamine by these enzymes are shown in Table 14.

As a result, the production of an extremely trace amount of L-alanyl-L-glutamine was observed even in the absence of enzyme, while a slight increase in the production rate was observed in the lot where carboxypeptidase or thiol endopeptidase was added as compared with the lot where no enzyme was added. In contrast, an overwhelmingly faster rate of production of L-alanyl-L-glutamine was observed in the lot where the present enzyme was added, and that rate of production was about 5,000 to 100,000 times faster than carboxypeptidase Y and thiol endopeptidase. As has been described above, the present enzyme was verified to have an extremely fast peptide production rate unlike any enzyme in the prior art. Furthermore, in contrast to the enzyme of the present invention being a dimer having a molecular weight of about 75,000, since the molecular weight of carboxypeptidase Y has been reported to be about 61,000, while that of thiol endopeptidase has been reported to be about 23,000 to 36,000, the L-alanyl-L-glutamine production rate per molecular weight is even greater for the enzyme of the present invention than that per unit weight indicated in the examples.

**Table 14**

| Enzyme | Amount of enzyme added (protein mg/ml) | L-Ala-L-Gln production rate (mM/min) | Ratio of L-Ala-L-Gln production rate per enzyme unit weight |
|---|---|---|---|
| No enzyme | 0 | 0.0006 | |
| Carboxypeptidase Y | 0.61 | 0.0257 | 0.0191 |
| Ficin | 2.60 | 0.0096 | 0.0017 |
| Papain | 2.30 | 0.0106 | 0.0021 |
| Bromelain | 2.80 | 0.0062 | 0.0010 |
| Chymopapain | 3.60 | 0.0100 | 0.0013 |
| Enzyme of present invention | 0.02 | 4.4000 | 100.0 |

### (Example 21) Production of L-Alanyl-L-Glutamine Using Microbial Cells of Sphingobacterium sp.

A 50 ml medium (pH 7.0) containing 5 g of glucose, 5 g of ammonium sulfate, 1 g of monopotassium phosphate, 3 g of dipotassium phosphate, 0.5 g of magnesium sulfate, 10 g of yeast extract and 10 g of peptone in 1 L was transferred to a 500 mL Sakaguchi flask and sterilized at 115°C for 15 minutes for culturing *Sphingobacterium sp.* strain FERM BP-8124 (Depositary institution: National Institute for Advanced Industrial Science and Technology, International Patent Organism Depositary, Address of depositary institution: Chuo Dai-6, 1-1 Higashi 1-Chome, Tsukuba-shi, Ibaraki-ken, Japan, International deposit date: July 22, 2002). This medium was then inoculated with one loopful of *Sphingobacterium sp.* strain FERM BP-8124 (Depositary institution: National Institute for Advanced Industrial Science and Technology, International Patent Organism Depositary, Address of depositary institution: Chuo Dai-6, 1-1 Higashi 1-Chome, Tsukuba-shi, Ibaraki-ken, Japan, International deposit date: July 22, 2002) cultured at 30°C for 24 hours in a slant agar medium (agar: 20 g/L, pH 7.0) containing 5 g of glucose, 10 g of yeast extract, 10 g of peptone and 5 g of NaCl in 1 L, followed by shake culturing at 30°C for 20 hours and 120 strokes/minute. A 1 ml aliquot of this culture broth was then added to the aforementioned medium (50 ml/500 mL Sakaguchi flask) and cultured at 30°C for 18 hours. Following completion of the culturing, the microbial cells were separated from the culture broths by centrifugation and suspended in 0.1 M borate buffer (pH 9.0) containing 10 mM EDTA to 100 g/L as wet microbial cells. A 0.1 mL aliquot of 100 mM borate buffer (pH 9.0) containing 10 mM EDTA, 200 mM L-alanyl methyl ester hydrochloride and 400 mM L-glutamine was then added to 0.1 mL of this microbial cell suspension, and after bringing to a final volume of 0.2 mL, was allowed to react at 25°C for 120 minutes. The amount of L-alanyl-L-glutamine produced at this time was 62 mM.

### (Example 22) Purification of Enzyme from Sphingobacterium sp.

The following procedure after centrifugal separation was carried out either on ice or at 4°C. *Sphingobacterium sp.* strain FERM BP-8124 (Depositary institution: National Institute for Advanced Industrial Science and Technology, International Patent Organism Depositary, Address of depositary institution: Chuo Dai-6, 1-1 Higashi 1-Chome, Tsukuba-shi, Ibaraki-ken, Japan, International deposit date: July 22, 2002) was cultured in the same manner as Example 21, and the microbial cells were collected by centrifugal separation (10,000 rpm, 15 minutes). After washing 2 g of microbial cells with 20 mM Tris-HCl buffer (pH 7.6), they were suspended in 8 ml of the same buffer and subjected to ultrasonic crushing treatment for 45 minutes at 195 W. This ultrasonic crushing liquid was then centrifuged (10,000 rpm, 30 minutes) to remove the crushed cell fragments and obtain an ultrasonic crushing liquid supernatant. This ultrasonic crushing liquid supernatant was dialyzed overnight against 20 mM Tris-HCl buffer (pH 7.6) followed by removal of the insoluble fraction by ultracentrifugation (50,000 rpm, 30 minutes) to obtain a soluble fraction in the form of the supernatant liquid. The resulting soluble fraction was applied to a Q-Sepharose HP column (manufactured by Amersham) pre-equilibrated with Tris-HCl buffer (pH 7.6), and the active fraction was collected from the non-adsorbed fraction. This active fraction was dialyzed overnight against 20 mM acetate buffer (pH 5.0) followed by removal of the insoluble fraction by centrifugal separation (10,000 rpm, 30 minutes) to obtain a dialyzed fraction in the form of the supernatant liquid. This dialyzed fraction was then applied to an SP-Sepharose HP column (manufactured by Amersham) pre-equilibrated with 20 mM acetate buffer (pH 5.0) to obtain the active fraction in which enzyme was eluted at a linear concentration gradient of the same buffer containing 0 to1 M NaCl.

### (Example 23) Production of L-Alanyl-L-Glutamine Using Enzyme Fraction

A 10 µl aliquot of the SP-Sepharose HP fraction (about 27 U/ml) purified in Example 22 was added to 90 µl of 111 mM borate buffer (pH 9.0) containing 111 mM L-alanine methyl ester hydrochloride, 222 mM L-glutamine and 11 mM EDTA, and allowed to react at 25°C for 120 minutes. As a result, 73 mM of L-alanyl-L-glutamine was produced in the enzyme-added lot. On the other hand, there was hardly any production of L-Ala-L-Glu observed in the enzyme-non-added lot, and the production amount was only about 0.07 mM after reacting for 120 minutes.

### (Example 24) Enzyme Substrate Specificity (11)

Substrate specificity was examined for enzyme derived from *Sphingobacterium sp.* strain FERM BP-8124 (Depositary institution: National Institute for Advanced Industrial Science and Technology, International Patent Organism Depositary, Address of depositary institution: Central. 6, 1-1-1 Higashi, Tsukuba City, Ibaraki Prefecture, Japan, International deposit date: July 22, 2002). 100 µl of 100 mM borate buffer (pH 9.0) containing the various carboxy components at a final concentration of 100 mM and the various amine components at a final concentration of 150 mM shown in Tables 15-1 to 15-4, the SP-Sepharose HP fraction enzyme purified in Example 22 (addition of 0.33 units in the reaction liquid) and 10 mM EDTA were allowed to react at 25°C for 1.5 hours. The amounts of each of the peptides produced in this reaction are shown in Table 15. (The "+" mark indicates those peptides for which production was confirmed but which were unable to be quantified due to the absence of a standard, while "tr" indicates a trace amount.) Furthermore, Tween-80 was added to the reaction system to a final concentration of 0.1% in the case of using L-Tyr-OMe. In addition, hydrochlorides were used for all carboxy components.

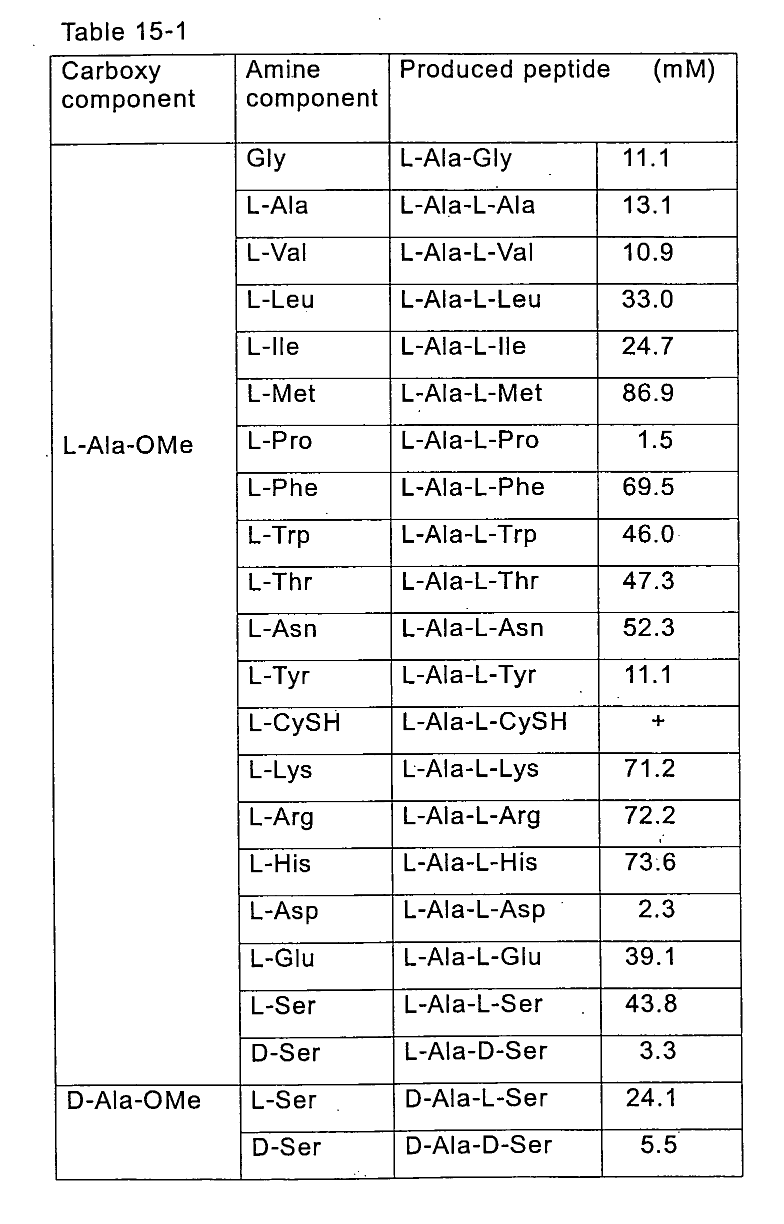

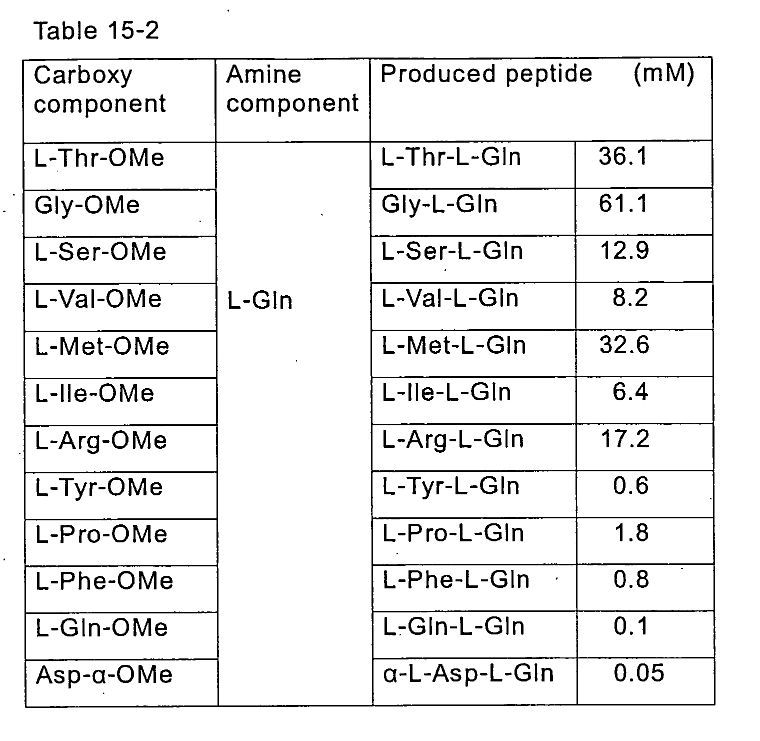

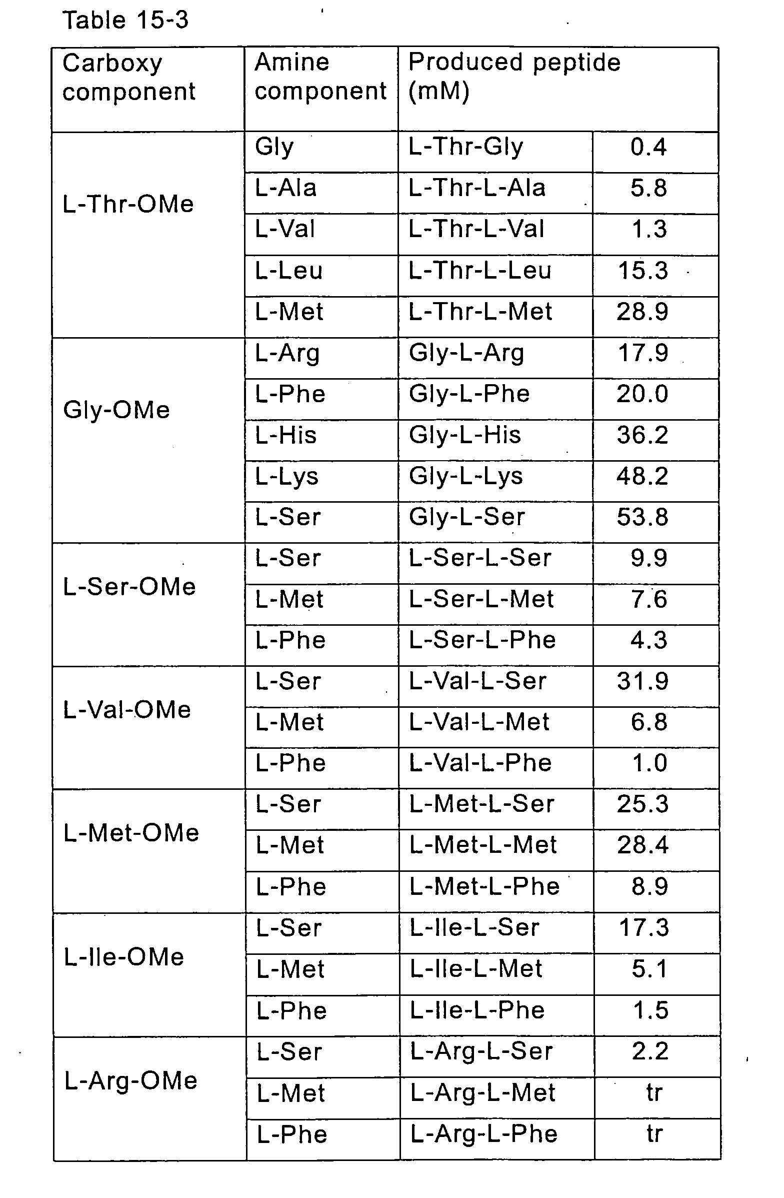

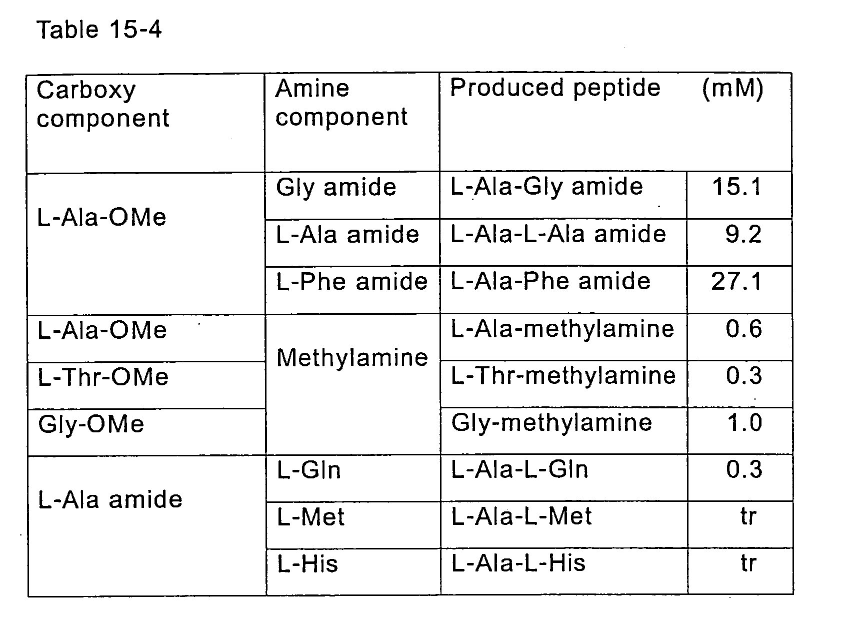

### (Example 25) Enzyme Substrate Specificity (12)

Substrate specificity with respect to oligopeptide production was examined for enzyme derived from *Sphingobacterium sp.* strain FERM BP-8124 (Depositary institution: National Institute for Advanced Industrial Science and Technology, International Patent Organism Depositary, Address of depositary institution: Chuo Dai-6, 1-1 Higashi 1-Chome, Tsukuba-shi, Ibaraki-ken, Japan, International deposit date: July 22, 2002). A 100 µl aliquot of 100 mM borate buffer (pH 9.0) containing the various carboxy components at a final concentration of 100 mM and the various amine components at a final concentration of 150 mM shown in Table 16, the SP-Sepharose HP fraction enzyme purified in Example 22 (addition of 0.33 units in the reaction liquid) and 10 mM EDTA were allowed to react for 1.5 hours at 25°C. The amounts of each oligopeptide produced in this reaction are shown in Table 18. Furthermore, hydrochloride salts were used for all carboxy components.

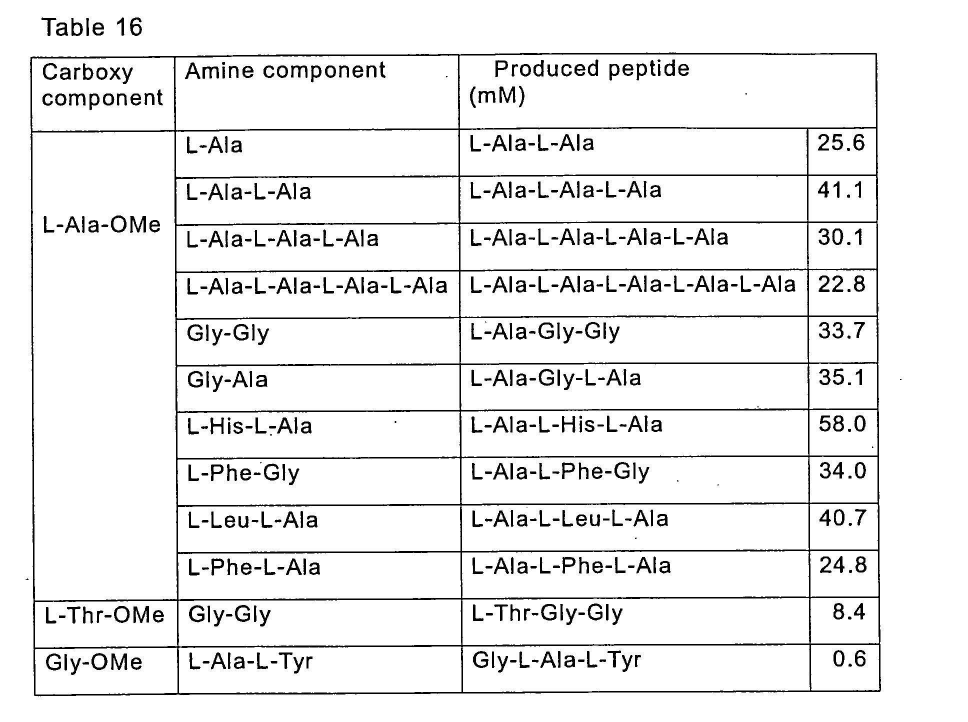

### (Example 26) Enzyme Substrate Specificity (13)

Substrate specificity was additionally assessed using the same enzyme fraction as that used in Example 5.

**Table 17**

| Carboxy component (mM) | | Amine component (mM) | | Produced peptide (mM) | | Reaction time (hr) |
|---|---|---|---|---|---|---|
| H-Ala-OMe | 50mM | H-p-F-Phe-OH | 50mM | H-Ala-p-F-Phe-OH | 21.9mM | 3 |
| H-Ala-OMe | 40mM | H-Cl-F-Phe-OH | 40mM | H-Ala-Cl-F-Phe-OH | 20.8mM | 3 |
| H-Ala-OMe | 40mM | H-p-NO₂-Phe-OH | 40mM | H-Ala-p-NO₂-Phe-OH | 27.5mM | 3 |
| H-Ala-OMe | 100mM | H-t-Leu-OH | 150mM | H-Ala-t-Leu-OH | 0.4mM | 3 |
| H-Ala-OMe | 20mM | H-2-Nal-OH | 20mM | H-Ala-2-Nal-OH | + | 3 |
| H-p-F-Phe-OMe | 100mM | H-Gln-OH | 150mM | H-p-F-Phe-H-Gln-OH | tr | 3 |
| H-Cl-F-Phe-OMe | 25mM | H-Gln-OH | 50mM | H-Cl-F-Phe-H-Gin-OH | tr | 3 |
| H-p-NO₂-Phe-OMe | 40mM | H-Gln-OH | 40mM | H-p-NO₂-Phe-H-Gln-OH | 1.1 mM | 3 |
| H-t-Leu-OMe | 100mM | H-Gln-OH | 150mM | H-t-Leu-H-Gln-OH | tr | 3 |
| H-2-Nal-OMe | 40mM | H-Gln-OH | 40mM | H-2-Nal-H-Gln-OH | tr | 3 |
| H-Aib-OMe | 100mM | H-Gln-OH | 150mM | H-Aib-H-Gln-OH | 18.8mM | 3 |
| H-N-Me-Ala-OMe | 100mM | H-Gln-OH | 150mM | H-N-Me-Ala-H-Gln-OH | 0.5mM | 3 |
| H-Aib-OMe | 100mM | H-Phe-OH | 150mM | H-Aib-Phe-OH | 17.2mM | 3 |
| H-CHA-OMe | 40mM | H-Phe-OH | 40mM | H-CHA-Phe-OH | + | 3 |
| H-N-Me-Ala-OMe | 100mM | H-Phe-OH | 150mM | H-N-Me-Ala-Phe-OH | tr | 3 |
| H-Ala-OMe | 100mM | H-Ser(tBu)-OH | 150mM | H-Ala-Ser(tBu)-OH | 48.8mM | 2 |
| H-Ser(tBu)-OMe | 100mM | H-Gln-OH | 150mM | H-Ser(tBu)-Gln-OH | tr | 2 |
| H-Ala-OMe | 100mM | H-Asp(OtBu)-OH | 150mM | H-Ala-Asp(OtBu)-OH | 62.6mM | 2 |
| H-Asp(OtBu)-OMe | 100mM | H-Gln-OH | 150mM | H-Asp(OtBu)-Gln-OH | 0.9mM | 2 |
| H-Ala-OMe | 100mM | H-Lys(Boc)-OH | 150mM | H-Ala-Lys(Boc)-OH | 51.0mM | 2 |
| H-Lys(Boc)-OMe | 100mM | H-Gln-OH | 150mM | H-Lys(Boc)-Gln-OH | + | 2 |

100 µl of reaction solutions consisting of 100 mM borate buffer (pH 9.0) containing each of the carboxy components and amine components at the final concentrations shown in Table 17, enzyme (addition of 0.1 unit in reaction solution) and 10 mM EDTA were allowed to react at 25°C for the reaction times shown in Table 17. The amounts of each of the peptides produced in the reactions are shown in Table 17. (A "+" mark indicates those for which production was confirmed but which were unable to be quantified due to the absence of a standard, while "tr" indicates a trace amount).

### (Abbreviations)

H-Ala-OMe: L-alanine methyl ester hydrochloride
H-p-F-Phe-OMe: p-fluoro-L-phenylalanine methyl ester hydrochloride
H-Cl-F-Phe-OMe: p-chloro-L-phenylalanine methyl ester hydrochloride
H-p-NO₂-Phe-OMe: p-nitro-L-phenylalanine methyl ester hydrochloride
H-t-Leu-OMe: tert-L-leucine methyl ester hydrochloride
H-2-Nal-OMe: 3-(2-naphthyl)-L-alanine methyl ester hydrochloride
H-Aib-OMe: α-aminoisobutyric acid methyl ester hydrochloride
H-N-Me-Ala-OMe: N-methyl-L-alanine methyl ester hydrochloride
H-CHA-OMe: β-cyclohexyl-L-alanine methyl ester hydrochloride
H-Ser(tBu)-OMe: O-tert-butyl-L-serine methyl ester hydrochloride
H-Asp(OtBu)-OMe: L-aspartic acid β-tert-butyl ester α-methyl ester hydrochloride
H-Lys(Boc)-OMe: N-ε-tert-butoxycarbonyl-L-lysine methyl ester hydrochloride
H-p-F-Phe-OH: p-fluoro-L-phenylalanine
H-Cl-F-Phe-OH: p-chloro-L-phenylalanine
H-p-NO₂-Phe-OH: p-nitro-L-phenylalanine
H-t-Leu-OH: tert-L-leucine
H-2-Nal-OH: 3-(2-naphthyl)-L-alanine
H-Gln-OH: L-glutamine
H-Phe-OH: L-phenylalanine
H-Ser(tBu)-OH: O-tert-butyl-L-serine
H-Asp(OtBu)-OH: L-aspartic acid β-tert-butyl ester
H-Lys(Boc)-OH: N-ε-tert-butoxycarbonyl-L-lysine

### (Example 27) Enzyme Substrate Specificity (14)

Substrate specificity with respect to oligopeptide production was assessed using the same enzyme fraction as Example 26. A 100 µl aliquot of reaction solutions consisting of 100 mM borate buffer (pH 9.0) containing each of the carboxy components and amine components at the final concentrations shown in Table 18, enzyme (the numbers of units added to the reaction solution are described in Table 18) and 10 mM EDTA were allowed to react at 25°C for 3 hours. The amounts of each of the oligopeptides produced in the reactions are shown in Table 18. (A "+" mark indicates those for which production was confirmed but which were unable to be quantified due to the absence of a standard, while "tr" indicates a trace amount). It should be noted that hydrochlorides were used for all of the carboxy components.

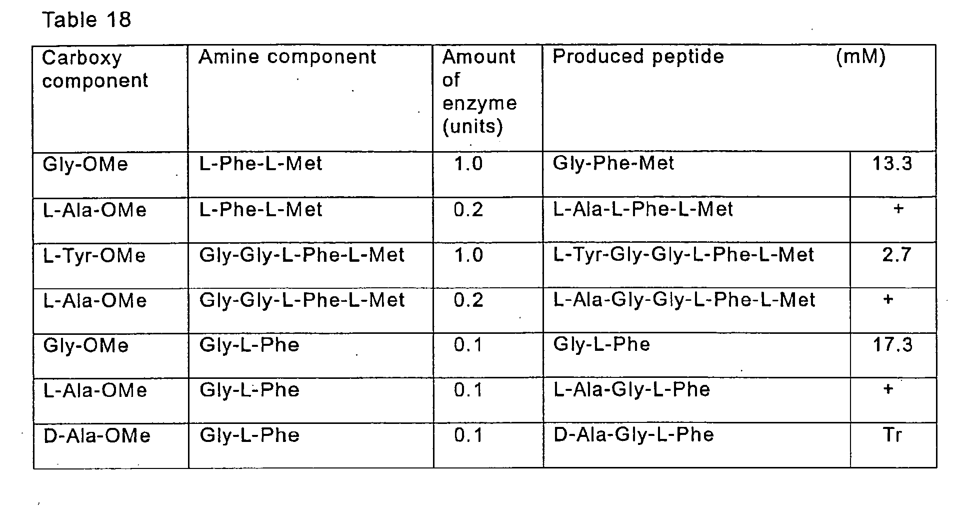

### INDUSTRIAL APPLICABILITY

The present invention provides a novel enzyme with which a peptide can be produced easily, inexpensively and at high yield by mitigating use of complex synthesis methods such as introduction and elimination of protecting groups. The use of the enzyme of the present invention enables efficient industrial production of peptides.

## Claims

1. An enzyme derived from the genus *Empedobacter* or the genus *Sphingobacterium,* and having the ability to produce a peptide from a carboxy component and an amine component.

2. An enzyme having the ability to produce a peptide from a carboxy component and an amine component and the ability to produce L-alanyl-L-glutamine at a production rate of 0.03 mM/min or more in a dipeptide-producing reaction under conditions (i) to (iv):
(i) the carboxy component is L-alanine methyl ester hydrochloride (100 mM);
(ii) the amine component is L-glutamine (200 mM);
(iii) the pH is 9.0; and
(iv) the amount of enzyme added is less than 0.61 mg/ml as protein amount.

3. The enzyme according to claim 1 or 2, wherein the carboxy component as a substrate includes both the amino acid ester and the amino acid amide.

4. The enzyme according to any one of claims 1 to 3, wherein any of an amino acid, a C-protected amino acid and an amine can be used as a substrate for the amine component.

5. The enzyme according to any one of claims 1 to 4, wherein the enzyme has the ability to produce a peptide within a pH range of 6.5 to 10.5.

6. The enzyme according to any one of claims 1 to 5, wherein the enzyme has the ability to produce a peptide within a temperature range of 0 to 60°C.

7. The enzyme according to any one of claims 1 to 6, wherein the enzyme is not inhibited by the serine enzyme inhibitor, phenylmethylsulfonyl fluoride, but is inhibited by p-nitrophenyl-p'-guanidinobenzoate.

8. The enzyme according to any one of claims 1 to 7, wherein the enzyme has a molecular weight as determined by SDS-gel electrophoresis of about 75 kilodalton, and a molecular weight as determined by gel filtration chromatography of about 150 kilodalton.

9. A microbe that produces an enzyme according to any one of claims 1 to 8.

10. The microbe according to claim 9, wherein the microbe is selected from *Empedobacter brevis* strain FERM BP-8113 and *Sphingobacterium sp.* strain FERM BP-8124.

11. A method for producing a dipeptide comprising producing a dipeptide from a carboxy component and an amine component using an enzyme according to any one of claims 1 to 8 or a substance containing the enzyme.
